(19) Europäisches Patentamt
European Patent Office
Office européen des brevets

(11) Publication number: **0 048 037 A1**

# (12) EUROPEAN PATENT APPLICATION

(21) Application number: 81109789.8

(22) Date of filing: 02.07.79

(51) Int. Cl.³: C 07 C 91/30
C 07 C 93/26, C 07 C 101/42
C 07 C 103/28, C 07 C 103/76

(30) Priority: 03.07.78 US 921670

(43) Date of publication of application: 24.03.82 Bulletin 82/12

(84) Designated Contracting States: DE GB IT NL SE

(60) Publication number of the earlier application in accordance with Art. 76 EPC: 0 007 204

(71) Applicant: ELI LILLY AND COMPANY
307, East McCarty Street
Indianapolis Indiana 46285(US)

(72) Inventor: Mills, Jack
7902 Timberhill Drive
Indianapolis Indiana 46217(US)

(72) Inventor: Schmiegel, Klaus Kurt
4507 Staughton Drive
Indianapolis Indiana 46226(US)

(72) Inventor: Shaw, Walter Norman
4511 North Broadway
Indianapolis Indiana 46205(US)

(74) Representative: Crowther, Terence Roger et al,
Erl Wood Manor
Windlesham Surrey GU20 6PH(GB)

## (54) Optically active 1-alkyl-3-(4-substituted-phenyl)-propylamines.

(57) Compounds of the formula (IV)

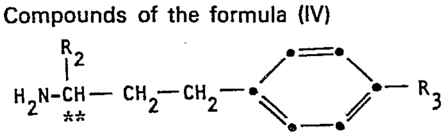

(IV)

wherein:

$R_2$ is methyl or ethyl

$R_3$ is hydroxy, $C_1$-$C_4$ alkanoyloxy, aminocarbonyl, methylaminocarbonyl or $C_1$-$C_2$ alkoxycarboxyl; and

$\underset{**}{C}$ is an asymmetric carbon atom having the S absolute stereochemical configuration.

The compounds are useful intermediates in the preparation of phenethanolamines having pharmaceutical properties.

EP 0 048 037 A1

OPTICALLY ACTIVE 1-ALKYL-3-(4-SUBSTITUTED-PHENYL)-PROPYLAMINES.

This invention relates to compounds useful as intermediates in the preparation of phenethanolamine derivatives. Such phenethanolamine derivatives are the subject of our co-pending application No. 79301279.0 publication No. 0007204.

In that application there are described compounds of the formula (I) :

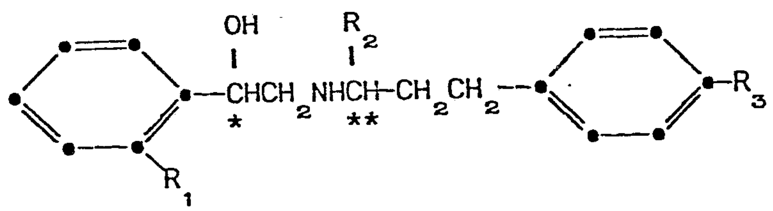

(I)

wherein :

$R_1$ is hydrogen or fluorine;

$R_2$ is methyl or ethyl;

$R_3$ is hydroxy, $C_1$-$C_4$ alkanoyloxy, aminocarbonyl, methylaminocarbonyl or $C_1$-$C_2$ alkoxycarbonyl;

$\underset{*}{C}$ is an asymmetric carbon atom having the R absolute stereochemical configuration; and

$\underset{**}{C}$ is an asymmetric carbon atom having the S absolute stereochemical configuration;

or a pharmaceutically-acceptable salt thereof.

Also in that application there is provided a pharmaceutical formulation which comprises a compound of formula (I), or a pharmaceutically-acceptable acid addition salt thereof, associated with a pharmaceutically-acceptable carrier therefor. A preferred formulation comprises a compound of the above formula wherein $R_2$ is methyl. An especially preferred pharmaceutical formulation comprises an R,S phenethanolamine of the above formula wherein $R_1$ is hydrogen, $R_2$ is methyl and $R_3$ is hydroxy or aminocarbonyl.

Whilst the compounds provided therein may be referred to generically as phenethanolamines, they will be systematically named herein as N-substituted-3-phenylpropylamines. The stereochemical configuration of the compounds is designated according to the R and S nomenclature. A full discussion of this system of nomenclature is presented by Cahn et al., Experientia, Vol. XII, pp. 81-124 (1956). The stereochemical configuration of the carbon atom labeled "C*" is R and will be presented first when naming the compounds The asymmetric carbon atom labeled "C**" has the S absolute stereochemical configuration. Such fact is designated in the systematic naming of such compounds as illustrated for the compound of the above formula wherein $R_1$ is hydrogen, $R_2$ is methyl and $R_3$ is aminocarbonyl: R,S-N-(2-phenyl-2-hydroxyethyl)-1-methyl-3-(4-aminocarbonylphenyl)propylamine.

The compounds provided in our co-pending application can be prepared by any of a number of methods involving well known and routinely used chemical processes. A preferred process involves the reaction of an optically active styrene oxide with an optically active 1-alkyl-3-phenylpropylamine.

That application also provides a method of preparing a compound of formula (I) in which a styrene oxide of formula (II):

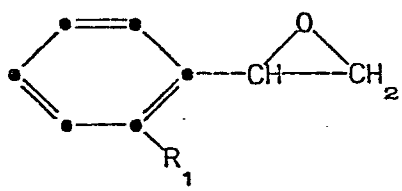 (II)

where $R_1$ is as defined above, is reacted with a propylamine derivative of formula (IV):

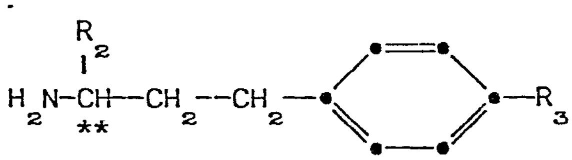 (IV)

where $R_2$ and $R_3$ are as defined above, followed in the case where the styrene oxide is not optically active, i.e. is not the R-styrene oxide, by resolution.

This invention relates to the optically active amines of formula (IV).

They can be prepared from the corresponding ketones of formula:

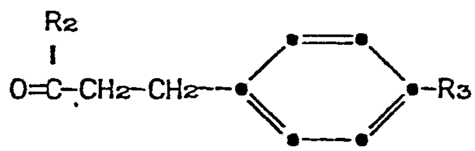

by reaction with (-)-α-methylbenzylamine in the presence of an acid such as p-toluenesulfonic acid to provide the corresponding S-N-(α-methylbenzyl)-1-($R_2$)-3-(4-($R_3$)phenyl)-propylimine. Reduction of this imine with Raney nickel gives

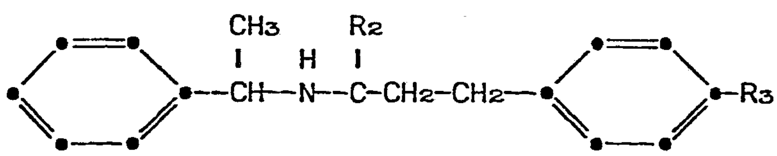

The above amine is salified, for example with HCl, and the salt purified by repeated crystallization from a suitable organic solvent. The optically active S,S-N-(α-methylbenzyl)-1-($R_2$)-3-(4-($R_3$)-phenyl)propylaminium salt thus obtained can then be hydrogenated with palladium on charcoal to remove the α-methylbenzyl group and liberate the amine of formula IV in salt form.

An optically active styrene oxide such as R-ortho-fluorostyrene oxide can be reacted with approximately an equimolar quantity of an optically active phenylpropylamine such as S-1-methyl-3-(4-methylaminocarbonylphenyl)propylamine to provide the corresponding optically active phenethanolamine, namely R,S-N-[2-fluorophenyl)-2-hydroxyethyl]-1-methyl-3-(4-methylaminocarbonylphenyl)propylamine. Condensation reactions of this type can be carried out in an unreactive polar organic solvent such as ethanol, dioxane, toluene or dimethylformamide, and usually at a temperature of from about 20 to about 120°C, preferably from 50 to 110°C. Under such conditions, the condensation generally is substantially complete after about 6 to about 10 hours, and the product phenethanolamine can be isolated by simply removing the reaction solvent, for instance by evaporation under reduced pressure. Further purification of the product thus formed can be accomplished if desired by standard methods including chromatography, crystallization and salt formation.

Another method for preparing the compounds of formula (I) comprises reacting a hydroxy protected 2-phenyl-2-hydroxyacetic acid acylating agent with an optically active 1-alkyl-3-phenylpropylamine, according to the invention, to provide an amide, followed by reduction of the amide carbonyl group to provide a compound of formula (I). This process may be depicted by the following reaction scheme:

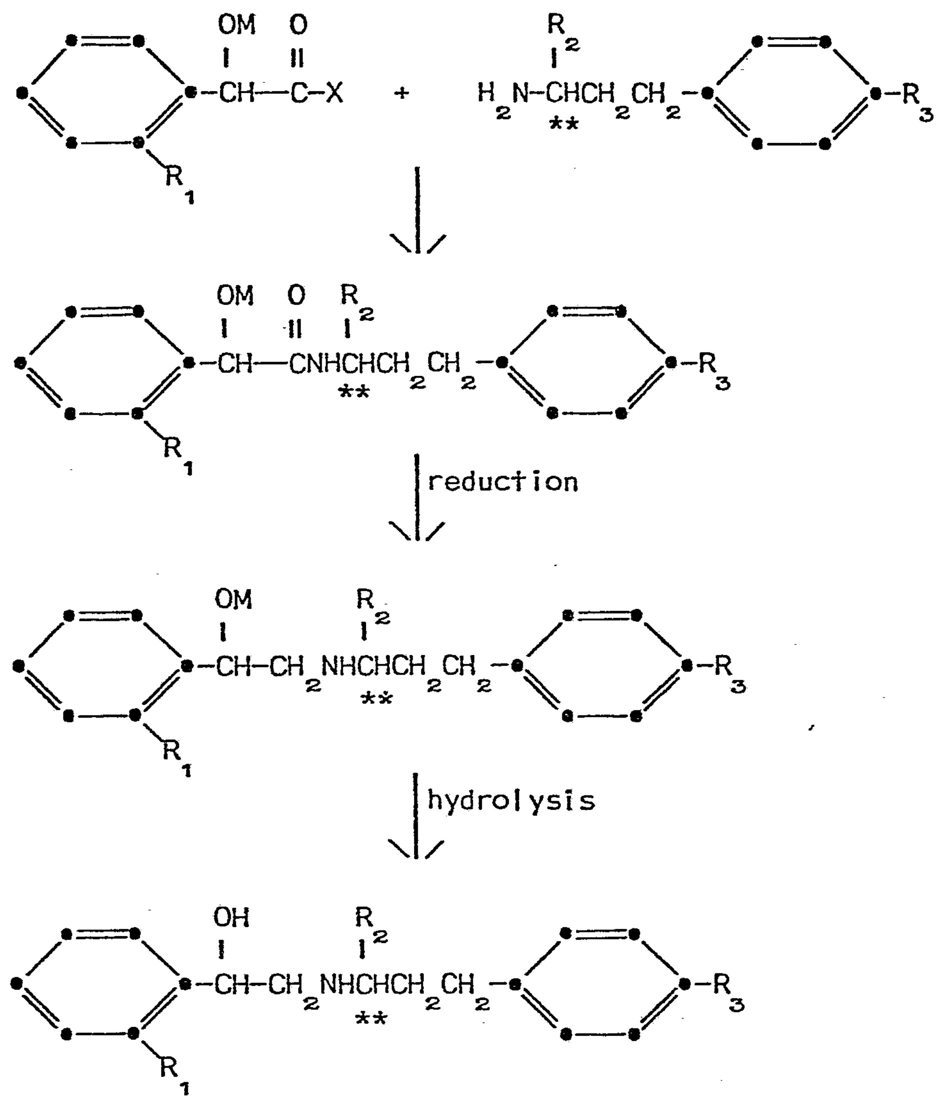


$R_1$, $R_2$ and $R_3$ in the above formulae are as defined above, X is a good leaving group and M is a readily removable hydroxy protecting group. Commonly used hydroxy protecting groups include acyl moieties such as acetyl, chloroacetyl and dichloroacetyl, as well as ether forming groups such as trimethylsilyl and the

like. Such readily removable hydroxy protecting groups are more fully described by E. Haslam in Protective Groups in Organic Chemistry, J.F.W. McOmie, Ed., Plenum Press, New York, N.Y., 1973, Chapter 3. As noted above, X is defined as a good leaving group and includes halogen radicals such as chloride and bromide, as well as acyloxy groups such as acetoxy and dichloroacetoxy. As an example of the preparation of a compound of formula (I) utilizing a hydroxy protected mandelic acid acylating agent, a compound such as R-2-(2-fluorophenyl)-2-hydroxyacetic acid can be reacted with dichloroacetyl chloride to provide R-2-(2-fluorophenyl)-2-dichloroacetoxyacetic acid. Reaction of this latter compound with a halogenating agent such as thionyl chloride or oxalyl chloride provides the corresponding acid chloride, namely R-2-(2-fluorophenyl)-2-dichloroacetoxyacetyl chloride. Reaction of this acid chloride with a phenylpropylamine, for instance S-1-methyl-3-(4-hydroxyphenyl)-propylamine, provides the corresponding amide, which amide in this case would be R,S-N-[2-(2-fluorophenyl)-2-dichloroacetoxy-1-oxoethyl]-1-methyl-3-(4-hydroxyphenyl)propylamine. The amide so formed is next reduced, for example with diborane, and the hydroxy protecting group is removed, for instance by hydrolysis, thus providing an optically active compound of formula (I).

A further method of preparing compounds of formula (I) comprises reacting a phenacyl halide with an optically active S-1-alkyl-3-phenylpropylamine.

For example, a phenacyl halide such as 2-fluorophenacyl bromide can be reacted with about an equimolar quantity of an amine such as S-1-methyl-3-(4-hydroxyphenyl)propylamine. Such alkylation reactions are normally carried out in an organic solvent such as ethanol and in the presence of a base such as sodium carbonate or triethylamine. The reaction is complete within about six hours when carried out at about 50°C. and provides a ketone intermediate, for instance S-N-[2-(2-fluorophenyl)-2-oxoethyl]-1-methyl-3-(4-hydroxyphenyl)propylamine. The ketone thus formed can be isolated as the free amine base or as an acid addition salt. Reduction of the ketone, for example with sodium borohydride, affords a compound of formula (I), generally as a mixture of optical isomers at C*. Such a mixture of R, S and S,S optical isomers can be separated by standard procedures, or if desired can be utilized directly since the S,S isomer is essentially devoid of biological activity.

Compounds having the above formula wherein $R_3$ is hydroxyl can be readily acylated to provide compounds in which $R_3$ is $C_1$-$C_4$ alkanoyloxy. As used herein, the term "$C_1$-$C_4$ alkanoyloxy" includes formyloxy, acetoxy, propionoxy, butyroxy, and isobutyroxy. The acylation of a compound of formula (I) having a phenolic hydroxyl group typically is accomplished by reacting

the hydroxyl compound with an acylating agent such as the appropriate acid anhydride or acid halide, for example the acid chloride or acid bromide. Typical acylating agents commonly employed include acetic anhydride, propionyl chloride, isobutyryl bromide and formic-acetic anhydride.

Compounds of the formula (I) wherein $R_3$ is aminocarbonyl can be prepared either directly by reacting the appropriate phenylpropylamine with a styrene oxide or alternatively can be derived from those compounds wherein $R_3$ is $C_1$-$C_2$ alkoxycarbonyl such as methoxycarbonyl or ethoxycarbonyl.

Compounds of formula (I) wherein $R_3$ is methylaminocarbonyl are preferably prepared by reacting a styrene oxide with a 3-(4-methylaminocarbonylphenyl)propylamine. The required phenylpropylamine starting material can be prepared by simply reacting methylamine with the appropriate benzoyl chloride derivative. For example, S-1-ethyl-3-(4-chlorocarbonylphenyl)propylaminium chloride can be reacted with methylamine to provide S-1-ethyl-3-(4-methylaminocarbonylphenyl)propylamine. Reaction of the latter compound with a styrene oxide affords a compound of formula (I).

In an effort to more fully illustrate particular aspects of this invention, the following Examples illustrate the preparation of compounds of formula (IV). The Examples are representative only and should not be construed as limiting in any respect.

EXAMPLE 1

Preparation of S-1-methyl-3-(4-aminocarbonyl-phenyl)propylamine

The oil from 280 g. of a 50% dispersion of sodium hydride in mineral oil was removed by several washings with hexane. The hexane was replaced with 1.5 l. of dimethylformamide, and the mixture was stirred and cooled in an ice-bath while 950 g. of ethyl acetoacetate was slowly added. After the sodium hydride had reacted a solution of 314 g. of α-bromo-p-toluic acid in 500 ml. of dimethylformamide was added. The ice bath was removed and the mixture was allowed to stir at 25°C. for eighteen hours, poured into water acidified with 3N hydrochloric acid and extracted with ethyl acetate. The combined organic layers were washed with water and brine and dried over anhydrous sodium sulfate. The solvent and excess ethyl acetoacetate were removed at reduced pressure to afford 500 g. of oil.

The crude oil was combined with 1.2 l. of glacial acetic acid, 140 ml. of concentrated hydrochloric acid and 750 ml. of water and heated at refluxed for three and one half hours. The solvents were removed under reduced pressure and the residue was dissolved in a mixture of ether and xylene and washed with water. The solid obtained after removal of the solvents was recrystallized twice from a mixture of ethanol and water to give 111 g. of 4-(4-carboxyphenyl)-2-butanone, m.p. 120.5-123°C.

Analysis calc. for $C_{11}H_{12}O_3$
Theory: C, 68.74; H, 6.29.
Found : C, 68.99; H, 6.10.

A mixture of 9.50 g. of 4-(4-carboxyphenyl)-2-butanone, 100 ml. of benzene, 15.7 g. of oxalyl chloride and one drop of dimethylformamide was stirred and warmed to 50°C. for two hours. The reaction mixture was then allowed to stir for eighteen hours at 25°C. and concentrated under reduced pressure. The residue was dissolved in 50 ml. of dioxane and, with stirring, was added slowly to 300 ml. of cold (0°C.) concentrated ammonium hydroxide. The mixture was stirred for seventy-five minutes and diluted to 1 l. with water. The solid was removed by filtration, washed with water, dried and recrystallized from a mixture of methanol and ether to provide 4.1 g. of 4-(4-aminocarbonylphenyl)-2-butanone, m.p. 144-146°C. An analytical sample, m.p. 149-150°C., was obtained by recrystallizing twice from a mixture of methanol and ether.

Analysis calc. for $C_{11}H_{13}NO_2$
Theory: C, 69.09; H, 6.85; N, 7.32.
Found : C, 68.98; H, 7.10; N, 6.99.

A solution of 45.7 g. of 4-(4-aminocarbonylphenyl)-2-butanone in 400 ml. of toluene containing 29.0 g. of (-)-α-methylbenzylamine and 1.0 g. of p-toluenesulfonic acid was heated at reflux for four hours with removal of water via a Dean Stark trap. After cooling the reaction mixture to room temperature, the solvent was removed therefrom by evaporation under reduced pressure to provide S-N-(α-methylbenzyl)-1-methyl-3-(4-aminocarbonylphenyl)-propylimine. The imine was hydrogenated in 625 ml. of methanol utilizing 15 g. of Raney Nickel

as the catalyst to provide a mixture of optical isomers of N-(α-methylbenzyl)-1-methyl-3-(4-aminocarbonylphenyl)propylamine. The amine was reacted with hydrogen chloride to provide the corresponding acid addition salt as a solid. The mixture was purified by repeated crystallization of the salt from methanol and acetonitrile to provide 23.6 g. of optically active S,S-N-(α-methylbenzyl)-1-methyl-3-(4-aminocarbonylphenyl)propylaminium chloride. M.P. 256-258°C. $[\alpha]_D$ -80.1° (MeOH).

A solution of 16.3 g. of the salt in 480 ml. of ethanol containing 3.0 g. of five percent palladium suspended on carbon was hydrogenated for twenty-two hours at 50°C. under a pressure of 60 p.s.i. The reaction mixture then was filtered and the solvent was removed from the filtrate by evaporation under reduced pressure to provide the product as a solid. The solid was recrystallized three times from ethanol and ethyl acetate to afford 9.4 g. of S-1-methyl-3-(4-aminocarbonylphenyl)propylaminium chloride. M.P. 237-240°C. $[\alpha]_D$ -8.2 (MeOH).

Analysis calc. for $C_{11}H_{17}ClN_2O$

Theory: C, 57.76; H, 7.49; N, 12.25

Found: C, 57.65; H, 7.57; N, 12.10

EXAMPLE 2

Preparation of S-1-methyl-3-(4-methylamino-carbonylphenyl)propylamine

Optically active S,S-N-(α-methylbenzyl)-1-methyl-3-(4-methoxycarbonylphenyl)propylamine was hydrolyzed by reaction with 10 percent aqueous hydrochloric acid to provide optically active S,S-N-(α-methylbenzyl)-1-methyl-3-(4-hydroxycarbonylphenyl)propylaminium chloride. M.P. 234-237°C. The acid thus formed was reacted with oxalyl chloride in benzene to provide the corresponding optically active acid chloride. The acid chloride was dissolved in dioxane and added dropwise to a stirred solution of methylamine in 50 ml. of dioxane. The reaction mixture was stirred for twelve hours at room temperature, and then diluted with 100 ml. of water. The aqueous reaction mixture was extracted with ethyl acetate, and the organic layer was separated, washed with water and dried. The solvent was removed by evaporation under reduced pressure to provide, after chromatography over silica gel, optically active S,S-N-(α-methylbenzyl)-1-methyl-3-(4-methylamino-carbonylphenyl)propylamine. Hydrogenation of the product thus formed according to the procedure of Preparation 3 effected de-benzylation to provide S-1-methyl-3-(4-methylaminocarbonylphenyl)propyl-aminium chloride. $[\alpha]_D$ -6° $[\alpha]_{365}$ -42.3° (MeOH).

EXAMPLE 3

S-1-Methyl-3-(4-aminocarbonylphenyl)propylamine

To 213 g. of (S)-(-)-α-methylbenzylamine in 1 l. of benzene was added 290 g. of 4-(4-hydroxyphenyl)-2-butanone and 0.5 g. of p-toluenesulfonic acid. The reaction mixture was stirred and heated to reflux. The water that was formed during the reaction was azeotropically removed. After the theoretical amount of water had separated, the benzene was removed at reduced pressure, and the residue was dissolved in methanol and hydrogenated in the presence of Raney nickel at ambient temperature over a sixteen hour period. The catalyst was removed by filtration, and the methanol and any unreacted α-methylbenzylamine were removed at reduced pressure. The residue was dissolved in 500 ml. of ethanol and an excess of hydrogen chloride in ether was added. The solid that formed was filtered, washed with ether, and recrystallized from a mixture of ethanol and acetone. Several more recrystallizations from a mixture of methanol and acetonitrile gave 263 g. of S,S-N-(α-methylbenzyl)-1-methyl-3-(4-aminocarbonylphenyl)propylamine, hydrochloride, m.p. 180-182°C. $[\alpha]_D$ -72.2, $[\alpha]_{365}$ -257.5° (MeOH).

A mixture of 258 g. of the above-named hydrochloride salt, 26 g. of 5 percent palladium on carbon and 1700 ml. of ethanol was hydrogenated at 60°C. for eight and one half hours. The catalyst was removed by filtration and the solvent was evaporated.

The residue was dissolved in hot ethanol, ether was added and 149 g. of crystalline title product was removed by filtration, m.p. 198-202°C., $[\alpha]_D$ -8.5, $[\alpha]_{365}$ -23.5° (MeOH).

CLAIMS

1. An optically active compound of formula (IV)

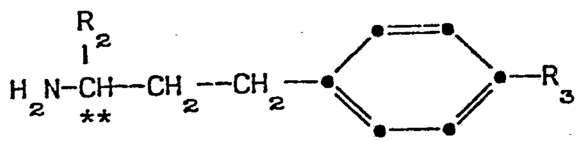

(IV)

wherein :

$R_2$ is methyl or ethyl;

$R_3$ is hydroxy, $C_1$-$C_4$ alkanoyloxy, aminocarbonyl, methylaminocarbonyl or $C_1$-$C_2$ alkoxycarboxyl; and

$C_{**}$ is an asymmetric carbon atom having the S absolute stereochemical configuration.

2. A compound according to claim 1 in which $R_2$ is methyl

3. A compound according to claim 1 or 2 in which $R_3$ is hydroxy, aminocarboxyl or methylaminocarboxyl.

European Patent Office

# EUROPEAN SEARCH REPORT

0048037

Application number

EP 81 10 9789

| Category | Citation of document with indication, where appropriate, of relevant passages | Relevant to claim | CLASSIFICATION OF THE APPLICATION (Int. Cl.³) |
|---|---|---|---|
| X | FR - A - 880 208 (TROPONWERKE DINKLAGE)<br>* examples 1,2 *<br>-- | 1-3 | C 07 C 91/30<br>93/26<br>101/42<br>103/28<br>103/76 |
| X | CHEMISCHES ZENTRALBLATT, volume 98, nr. 10, March 9, 1927<br>BERLIN (DE)<br>C.MANNICH et al.: "Uber einige vom 1-Phenyl-3-Aminobutan sich ableitende Phenolbasen"<br>pages 1442,1443<br>& Arch.der Pharm..u.Ber.der Pharm. Ges.265. 15-26<br>* the entire abstract *<br>-- | 1-3 | TECHNICAL FIELDS SEARCHED (Int. Cl.³)<br>C 07 C 91/30<br>93/26<br>103/28<br>103/76 |
| A | FR - A - 2 199 458 (F.HOFFMANN LA ROCHE& CIE.S.A.)<br>* page 1, lines 1-21 *<br>---------- | 1-3 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| THE HAGUE | 17-12-1981 | PAUWELS |

EPO Form 1503.1 06.78